# EUROPÄISCHE PATENTSCHRIFT

(11) **EP 3 576 805 B1**
(45) Veröffentlichungstag und Bekanntmachung des Hinweises auf die Patenterteilung: **15.11.2023**
(21) Anmeldenummer: 18710297.5
(22) Anmeldetag: 01.02.2018
(51) Int. Cl.: A61M 60/205, A61M 60/50

(54) **ANORDNUNG MIT EINER BLUTPUMPE, EINER STEUEREINHEIT UND EINEM GERÄT ZUR ÜBERMITTLUNG DER MESSWERTE**
ARRANGEMENT WITH A BLOOD PUMP, A CONTROL UNIT AND A DEVICE FOR TRANSMITTING THE MEASURED VALUES
ENSEMBLE COMPRENANT UNE POMPE À SANG, UNE UNITÉ DE COMMANDE ET UN APPAREIL DE TRANSMISSION DE VALEURS MESURÉES

(30) Priorität: 02.02.2017 DE 102017000940
(43) Veröffentlichungstag der Anmeldung: 11.12.2019
(73) Patentinhaber: Xenios AG, 74076 Heilbronn (DE)
(72) Erfinder: MATHEIS, Georg, 74076 Heilbronn (DE); KÖHLER, Sebastian, 74354 Besigheim (DE)
(74) Vertreter: Graf von Stosch, Andreas
(86) Internationale Anmeldenummer: PCT/DE2018/000019
(87) Internationale Veröffentlichungsnummer: WO 2018/141323

(56) Entgegenhaltungen:
- WO-A1-2018/073150
- DE-A1-102014 107 980
- US-A- 3 206 768
- US-A1- 2011 054 239
- US-B2- 7 988 728

## Beschreibung

Die Erfindung betrifft eine Anordnung zur extrakorporalen Lungen- und Herzunterstützung mit einer Blutpumpe, einem Gasaustauscher, einer Steuereinheit, um den Durchfluss an der Blutpumpe zu steuern und den optimalen Zeitpunkt der Blutentnahme an einem Patienten in Abhängigkeit von der Atmung des Patienten abzupassen und den Blutfluss entsprechend einzustellen, einer Kanüle, mit der vom Patienten Blut entnommen werden kann, das mit der Blutpumpe durch den Gasaustauscher gefördert wird, wobei die Kanüle mit der Blutpumpe in Verbindung steht, sodass Blut mit der Blutpumpe durch den Gasaustauscher gefördert werden kann, und mindestens einem Gerät, um Messwerte an die Steuereinheit zu übermitteln.

Bei der extrakorporalen Lungen- und Herzunterstützung (ECLA / ECLS bzw. ECMO, ECCO2R) ist der Zielblutfluss von verschiedenen Faktoren (I<anülengröße, Eigenschaften der Membranlunge, Intravasales Blutvolumen und dem Verhältnis von Gefäß- und Kanülendurchmesser) abhängig. Dabei kann es bei der Blutentnahme bzw. Drainage dazu kommen, dass sich die Öffnungen der Kanüle an der Gefäßwand festsaugen.

Um Blut bei den genannten Verfahren Blut dem Körper zuzuführen werden Kanülen verwendet, die einen ausreichenden Blutfluss garantieren. Hierfür werden je nach Anwendung Kanülen mit einem äußeren Durchmesser von ca. 12 - 32 Fr (French) und eine Länge von ca. 90 bis 800 mm verwendet.

Im Hinblick auf diese Kanülen unterscheidet man zwischen Singlelumen- und Doppellumenkanülen. Beispiele hierfür sind die NovaPort^{®} twin Doppellumenkanüle von Novalung^{®} oder Medos^{®} Femoralkanüle.

Zur Drainage wird die Kanüle in den meisten Fällen bis vor das Herz bzw. in die Hohlvene, oder sogar ins rechte Atrium geschoben. Typische Kanülierungsorte zur Drainage sind die vena femoralis, vena jugularis oder vena subclavia. Die Kanülenspitze bzw. der Drainagebereich der Kanüle liegt somit oft im Bereich des Brustkorbes.

In Folge der Ausdehnung des Innenvolumens des Brustkorbes kann sich bei Spontanatmung und Beatmung die Füllung der Blutgefäße bzw. das Blutvolumen in den Gefäßen im Brustkorb und der Peripherie im Atemzyklus ändern. Die Kanüle wird dann trocken gelegt bzw. saugt sich an der Gefäßwand an, da zu bestimmten Phasen des Atemzyklus weniger Blutvolumen vorhanden ist als Patientendruck (Blutfluss des Patienten).

Bei der Beatmung wird die Lunge ständig einem positiven Druck ausgesetzt, sowohl während der Inspiration und Expiration. Bei der Inspiration unter Spontanatmung ist der Druck in der Lunge hingegen zeitweilig geringer als der Umgebungsdruck. Es gibt somit zwei zu unterscheidende Arten der Atmung.

Eine gattungsgemäße Anordnung zeigt die EP 2 832 383 A1, bei der einem Patienten Blut pulsatil und synchronisiert zum Herzen zurückgegeben wird. Dabei wirkt eine Steuereinheit auf eine Blutpumpe und ein EKG stellt ein Steuersignal für die Steuereinheit zur Verfügung, damit die Steuereinheit entsprechend den EKG-Werten den Durchfluss an der Blutpumpe steuern kann.

Die EP 2 832 383 A1 beschreibt somit ein EKG-getriggertes Pumpverfahren, bei dem Blut beispielsweise aus einer ECLS-Anordnung passend zur richtigen Herzphase in den Patienten zurück gepumpt wird. Die Pumpe sorgt für spezielle Pulsspitzen, um das Herz zu unterstützen. Dabei entsteht das Problem, dass der für die Herzunterstützung benötigte Volumenstrom auch aus dem Patienten entnommen werden muss und sogar zu Volumenstromspitzen an der Blutentnahmekanüle führen kann. Dadurch wird das Problem des Festsaugens einer Kanüle an der Gefäßwand verstärkt.

Dieses Problem kann durch eine geeignete Formgebung an der Kanüle oder durch ein Ausgleichsgefäß im Blutkreislauf vermindert werden.

Die EP 2 117 623 B1 schlägt vor, Werte zum Blutfluss zu ermitteln, um die Drehzahl einer Blutpumpe kurz vor einem Gefäßkollaps schrittweise zu erhöhen und zu drosseln. Dadurch kann kurzzeitig auf Probleme des Blutflusses reagiert werden.

Die US 2011/054239 A1 offenbart ein System zur Steuerung der Geschwindigkeit einer rotierenden Blutpumpe, wobei das System die Geschwindigkeit der Pumpe oszilliert, um einen pulsierenden Druck am Auslass der Pumpe zu erzeugen. Der natürliche Herzzyklus wird mit Hilfe eines Pulsatilitätsindexes bestimmt und die Drehzahl wird synchron mit dem natürlichen Herzzyklus oszilliert.

Die DE 10 2014 107980 A1 bezieht sich auf ein Beatmungsystem zur Unterstützung des Blutgasaustauschs mittels maschineller Beatmung und extrakorporalem Blutgasaustausch, das dazu ausgebildet ist, eine maschinelle Atmungsunterstützung durch die Beatmungseinrichtung einerseits und einen extrakorporalen Blutgasaustausch durch die ECLS-Einrichtung andererseits in koordinierter Weise automatisiert durchzuführen. Dabei gibt die ECLS-Einrichtung ein Niveau des extrakorporalen Blutgasaustauschs vor und die Beatmungseinrichtung stellt sich anhand des von der ECLS-Einrichtung vorgegebenen Niveaus des extrakorporalen Blutgasaustauschs automatisiert auf ein Niveau der maschinellen Atmungsunterstützung ein.

Die US 3 206 768 A offenbart eine Vorrichtung zum Zuführen von Blut zu mindestens einem Teil des Kreislaufsystems mit einer elektromagnetische Pumpe, einem Mittel zur Erzeugung eines Signals, das den aktuellen Blutdruck anzeigt, einem Mittel zur Erzeugung eines Referenzsignals, das den gewünschten Blutdruck angibt, einem Mittel zum Vergleich des tatsächlichen Blutdrucksignals mit dem Referenzsignal, um ein Fehlersignal zu erzeugen, einem Mittel, die das Fehlersignal zu den Pumpenbetätigungsmitteln leiten, um die Rate der Pumpe zu verändern und das Fehlersignal zu eliminieren, und einem Mittel zur Änderung des Referenzsignals in Reaktion auf Änderungen der Atmungsrate des Körpers.

Die meisten Lungen- und Herzunterstützungsverfahren nutzen jedoch einen kontinuierlichen Blutfluss. Hierbei wird die Drehzahl der Blutpumpe oder der Zielblutfluss manuell eingestellt, um den Blutfluss festzulegen. Ein ECMO-System wird beispielsweise durch die Messung von drei Druckmesspunkten im extrakorporalen Kreislauf oder die Messung der Druckdifferenz zwischen den Messstellen kontrolliert. Dadurch kann bei einem Druckanstieg der Ort des Problems leichter lokalisiert werden. Außerdem können an der Steuereinheit Schwellenwerte eingestellt werden, bei denen ein Alarm ausgelöst wird.

Der Erfindung liegt die Aufgabe zugrunde, einen möglichst sicheren, angepassten und schonenden Blutfluss zu ermöglichen. Außerdem soll das Problem des Ansaugens einer Kanüle an einer Gefäßwand bei konstantem Blutfluss und insbesondere auch bei sich änderndem Blutvolumenstrom verringert werden.

Diese Aufgabe wird mit einer Anordnung gemäß dem unabhängigen Anspruch 1 gelöst, bei der das Gerät zur Abgabe eines Parameters des Atemzyklus oder mit dem Atemzyklus verbundenen Parameters ausgebildet ist. Dadurch kann der größtmögliche an den Gefäßzustand adaptierte Blutfluss bereitgestellt werden.

Erfindungsgemäß wird eine intelligente, automatisierte und verbesserte Blutpumpensteuerung bereitgestellt, welche durch den Atemzyklus oder einen damit verbundenen Parameter getriggert wird. Dies ermöglicht es, die optimalen Zeitpunkte für eine Erhöhung oder Absenkung der Pumpenleistung zu ermitteln, um das für den extrakorporalen Kreislauf erforderliche Blutvolumen blutschonend und mit einer möglichst kleinen Kanüle zu fördern.

Dies ermöglicht es, nicht nur im Rahmen von Extremfällen, sondern möglichst kontinuierlich den Durchfluss an der Blutpumpe auf die aktuellen Patientenbedingungen (d. h. den im Atemzyklus wechselnden Füllungszustand der großen Venen) abgestimmt zu steuern, um mit der Drainage verbundene Probleme präventiv zu vermeiden. Dabei sollten Blutflüsse bis zu 5 und vorzugsweise bis zu 8 l/min erreicht werden, ohne ein Ansaugen der Kanüle an der Gefäßwand befürchten zu müssen. Die atemgetriggerte Drainage ermöglicht es, den optimalen Zeitpunkt der Blutentnahme am Patienten in Abhängigkeit von der Atmung des Patienten abzupassen und den Blutvolumenstrom passend einzustellen. Hierdurch wird ein schonender Blutfluss ermöglicht und ein Ansaugen der Kanüle an der Gefäßwand verhindert.

Mit der erfindungsgemäßen Anordnung kann die Drainage verbessert werden, während das Risiko von Drainageproblemen minimiert wird. Dies ermöglicht es, eine optimale Versorgung des Patienten bereitzustellen. Da die Sensoren zur Ermittlung der Parameter verfügbar sind, ist eine derartige Anordnung leicht zusammenzustellen und direkt patientenadaptiert einsetzbar. Die vorgeschlagene Lösung ist somit eine einfache aber sehr effektive Lösung, um ein Ansaugen der Kanüle an einer Gefäßwand zu vermeiden und einen besonders hohen Blutvolumenstrom zu ermöglichen. Dies ermöglicht eine besonders sichere Behandlung des Patienten, da ein kontinuierlicher und hoher Blutfluss die Effektivität von Gasaustauschsystemen garantiert.

Die Signaleingabe für diese Triggerung kann - aber muss nicht - ein EKG sein. Im klinischen Einsatz wird darauf geachtet, ob der Patient künstlich, also mechanisch, beatmet wird. Dies ist wichtig, um bestimmen zu können, ob die Leistung der Blutpumpe, wie beispielsweise einer Rollerpumpe oder einer Axial-, Radial oder Diagonalpumpe erhöht oder gedrosselt wird.

Als Teil der Anordnung eignet sich eine Blutpumpe mit einem Antrieb, dessen Leistung zur Variation des Durchflusses an der Blutpumpe gesteuert werden kann.

Vorteilhaft ist es, wenn die Blutpumpe einen Rotor aufweist, um zur Variation des Durchflusses dessen Drehzahl zu steuern.

Die Anordnung weist einen Gasaustauscher auf, der das Patientenblut decarboxyliert (CO₂ entfernt) und wenn notwendig auch oxygeniert (mit O₂ anreichert).

Eine vorteilhafte Ausführungsvariante sieht vor, dass das Gerät ein EKG ist und der Parameter mit einer Impedanz des EKG korreliert. Ein EKG kann auch für einen beatmeten Patienten genutzt werden. Besonders vorteilhaft ist jedoch der Einsatz eines EKG für einen spontan atmenden Patienten.

Um die entsprechenden Triggerpunkte zu identifizieren, können die bereits in der Praxis verwendeten sensorischen Daten benutzt werden. So kann beispielsweise die Atemfrequenz bei einem spontan atmenden Patienten über die Impedanz des EKG, insbesondere über das EKG Monitoring-Kabel an die Steuereinheit übermittelt werden.

Alternativ oder kumulativ wird vorgeschlagen, dass das Gerät ein Ventilationsgerät ist und der Parameter mit einem Inspirationsdruck des Ventilationsgerätes korreliert. Dann wird das Gerät vor allen anderen sensorischen Daten priorisiert und als Triggersignal verwendet. Der Inspirationsdruck wird als kontinuierliches Druck-Zeit-Diagramm dargestellt, dessen Extrempunkte oder Plateauphasen mit optional einer zeitlichen Abweichung als Triggersignal oder Triggerpunkt verwendet werden, um als Parameter an die Steuereinheit übermittelt zu werden. Somit können insbesondere bei einem beatmeten Patienten die Parameter durch das Ventilationsgerät gesteuert werden.

Weiterhin wird vorgeschlagen, dass das Gerät ein Brustgurt sein kann. Der Parameter korreliert dann mit einer Dehnung oder Spannung am Brustgurt.

In entsprechender Weise kann das Gerät auch ein Piezoelement sein.

Als weitere Ausführungsvariante des Gerätes wird ein Zwerchfellsensor oder ein Myokardsensor vorgeschlagen. Ein Myokardsensor ist ein EMG-Sensor (Elektromyografiesensor), der die elektrische Muskelaktivität, das heißt die Aktionspotentiale der Muskeln misst. Damit kann beispielsweise die Aktivität der Atemmuskulatur gemessen werden, um die Messwerte (z.B. Peaks) als Triggerpunkt an die Steuereinheit zu übermitteln.

Außerdem kann das Gerät auch eine sensorische Magensonde sein, die Messwerte an die Steuereinheit übermittelt.

Es ist vorteilhaft, wenn möglichst viele sensorische Daten an die Steuereinheit übermittelt werden. Dies ermöglicht es, mit der Steuereinheit Blutfluss- und Gasflussparameter eines Gasaustauschers und ein Ventilationsgerät zu überwachen und insbesondere das Ventilationsgerät und gegebenenfalls auch die Temperierung für den Gasaustauscher mit der Steuereinheit zu regeln und zumindest die relevanten Parameter an der Steuereinheit einzugeben.

Besonders vorteilhaft ist eine Hybridlösung sowohl für die extrakorporale Lungen- oder Herzunterstützung als auch für die Ventilation des Patienten mittels einer für beide Verfahren verwendbaren Steuereinheit, die automatisiert die Blutfluss- und Gasflussparameter für das Ventilationsgerät steuert und eine parallele intelligente Beatmung bereitstellt.

Ob ein Patient spontan atmet oder beatmet ist, spielt eine entscheidende Rolle. Bei einer Spontanatmung sollte beim Einatmen die Drehzahl reduziert und beim Ausatmen erhöht werden. Bei der Beatmung ist das Prinzip umgekehrt. Die beschriebene Anordnung ermöglicht es, zwischen diesen beiden Stadien zu unterscheiden und automatisch die aktuelle Situation zu erkennen, um den Durchfluss an der Blutpumpe entsprechend zu steuern. Dies wird erfindungsgemäß durch die bereitgestellten Schnittstellen ermöglicht.

Aus einer erhöhten Blutentnahme zu einem bestimmten Zeitintervall des Atmungszyklus resultiert entsprechend auch eine zeitlich erhöhte Rückgabe von Blutvolumen. Dies kann beispielsweise dann zu Problemen führen (z.B. Rezirkulation bzw. Shunt), wenn eine venovenöse Kanülierung wie z.B. eine venovenöse Doppellumenkanüle eingesetzt wird, da dabei die Entnahme und Rückgabe regional sehr nah beieinander liegen. Daher sind die Vorteile der Erfindung auch besonders bei venöser Entnahme und arterieller Rückgabe des Blutes ausgeprägt.

Die Steuereinheit kann auf der Grundlage von vorher eingegebenen Daten Messwerte von unterschiedlichen Geräten auswählen oder kombinieren, um den Durchfluss an der Blutpumpe zu steuern. Dies ermöglicht es, dass die Steuereinheit Signale aus verschiedenen Geräteeingängen auswählt oder ein kombiniertes Triggersignal erstellt.

In der Praxis kann die Steuereinheit als Vorrichtung den Signaleingang der verschiedenen "Geräte" durch ein leitendes Signal bzw. einen Kontakt, alternativ auch eine mechanische Konnektierung (einfacher Schalter) erkennen. Es gibt mehrere Konnektionsstellen bzw. Buchsen in einer Steuereinheit. Bei der mech. Beatmung gibt es zwangsweise ein Ventilationsgerät und somit einen Signaleingang. Hierfür kann die Steuereinheit eine separate Buchse / Port haben, welcher nur für das Ventilationsgerät bzw. den entsprechenden Stecker geeignet ist und auch so gekennzeichnet ist. Wenn diese Buchse belegt ist und das Signal verwendet wird, wird die Blutpumpe entsprechend mit der Steuerung für eine mechanische Beatmung betrieben.

Das Eingangssignal wird in der Praxis von der Steuereinheit mit einem Algorithmus "behandelt". Dieser fragt dauerhaft alle Signaleingänge ab und prüft regelmäßig auf fehlerhafte Signale. Hierzu dient ein Speicher. Auf diesem sind die notwendigen Parameter eines jeden Eingangssignals und die dazugehörigen maximalen Abweichungen vermerkt. Außerdem kann ein eventuelles "Rauschen" des Eingangssignals vorher mit einem zusätzlichen Algorithmus bzw. einem mathematischen Verfahrens (bspw. Laplace) herausgerechnet werden, um Ungenauigkeiten zu vermeiden.

Alle geprüften Signale werden von der Steuereinheit bewertet bzw. priorisiert. Es muss mindestens ein geprüftes Signal (vom "Gerät") vorhanden sein. Gibt es mehrere Signaleingänge wird das höher gelistete (im Speicher) verwendet / priorisiert. Zusätzliche Signaleingänge dienen mit Hilfe eines weiteren Speichers dazu, den Aktivierungspunkt (Signal an die Blutpumpe (wann genau Leistung runter oder rauf) zu präzisieren, indem die Werte live bspw. in einer CPU oder einem RAM-Speicher gegengerechnet werden. Zusammen mit weiteren Parametern zur Verzögerung kann somit ein optimales Timing für die Blutpumpe errechnet werden.

Ob es sich um ein Signal für einen spontan atmenden, oder mechanisch beatmeten Patienten handelt kann außerdem leicht automatisch (ohne manuelle Einstellungen vom Arzt) von der Steuereinheit berechnet werden. Hierzu werden entsprechend auch verschiedene Parameter (bspw. Druck) mit Vorgaben aus dem Speicher abgeglichen. Siehe Absatz [07] oben.

Um insbesondere für eine venovenöse Kanülierung ein Delay in den Kreislauf einzufügen, das heißt um das Blut versetzt von der Entnahme zurückzugeben, wird vorgeschlagen, dass nach der Blutpumpe ein Pufferelement angeordnet ist. Das Pufferelement kann beispielsweise ein flexibler kleiner Stauraum oder ein Reservoir sein. Weitere Möglichkeiten erschließen sich durch eine Doppelpumpenvariante oder eine ventilgesteuerte Rückgabe.

Die Erfindung wird im Folgenden anhand der Zeichnung näher erläutert. Es zeigt
- Figur 1: schematisch die Behandlung eines Patienten auf einer Intensivstation nach dem Stand der Technik,
- Figur 2: schematisch die Behandlung eines Patienten auf der Intensivstation mit sensorischem Input und Schnittstelle zur Beatmung,
- Figur 3: schematisch die Behandlung eines Patienten auf einer Intensivstation mit einer gemeinsamen Steuereinheit und,
- Figur 4: schematisch eine Doppellumenkanüle im Schnitt.

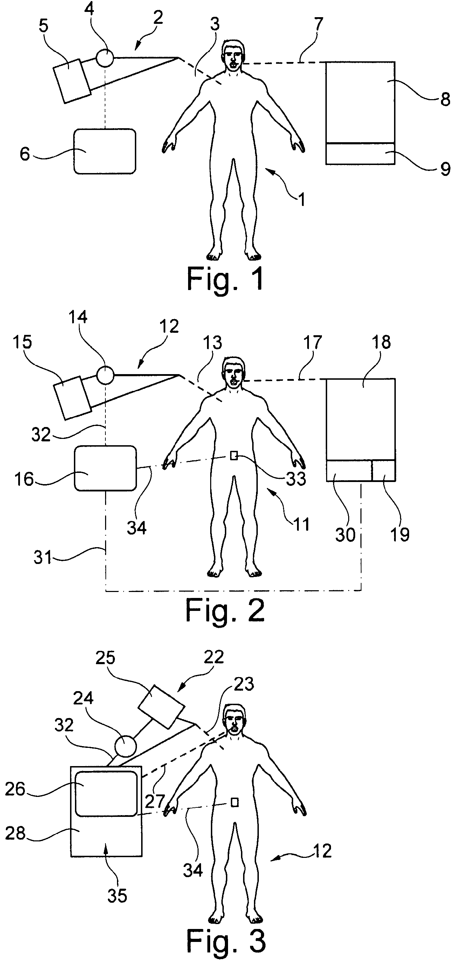

Der in Figur 1 gezeigte Patient 1 ist mit einer Anordnung 2 über eine Kanüle 3 verbunden. Die Anordnung 2 weist eine Blutpumpe 4 und einen Gasaustauscher 5 auf. Die Blutpumpe 4 ist mit einer Steuereinheit 6 verbunden, die den Durchfluss an der Blutpumpe 4 steuert. Die Kanüle 3 ermöglicht es als Doppellumenkanüle im gleichen Gefäß Blut zu entnehmen und Blut zuzuführen. Dabei wird durch die Ausbildung und Anordnung der Kanüle darauf geachtet, dass das zugeführte Blut nicht sofort wieder entnommen wird. Dies führt dazu, dass im Bereich der Blutentnahme die Gefäßwand an die Kanüle gezogen werden kann, wenn nicht schnell genug Blut nachfließen kann. Dies führt dazu, dass die Ansaugöffnungen in der Kanüle durch die Gefäßwand verschlossen werden und das Gefäß kollabiert. Die Steuereinheit kann dazu dienen, Blut passend zur richtigen Herzphase zurück in den Patienten zu pumpen. Im Folgenden wird gezeigt, wie es die Steuereinheit ermöglicht, den optimalen Zeitpunkt der Blutentnahme am Patienten in Abhängigkeit von der Atmung des Patienten abzupassen und den Blutfluss entsprechend einzustellen.

Daher ist der Patient 1 über die Leitung 7 mit einem Ventilationsgerät 8 verbunden, das eine Beatmungskonsole 9 aufweist.

Die in Figur 2 gezeigte Anordnung 12 dient der Behandlung des Patienten 11, der über eine Kanüle 13 mit einer Blutpumpe 14 in Verbindung steht. Die Kanüle 13 ist wie die Kanüle 3 eine Doppellumenkanüle, mit der vom Patienten 11 Blut entnommen werden kann, das mittels der Blutpumpe 14 durch den Gasaustauscher 15 gefördert wird. Die Blutpumpe 14 wird über die Steuereinheit 16 gesteuert.

Außerdem steht der Patient 11 über die Leitung 17 mit dem Ventilationsgerät 18 in Verbindung, das eine Beatmungskonsole 19 aufweist.

Diese Beatmungskonsole 19 des Ventilationsgerätes 18 weist eine Einheit 30 auf, die über die Ventilationsgeräteschnittstelle 31 mit der Steuereinheit 16 in Verbindung steht. Außerdem steht die Steuereinheit 16 über die Pumpenschnittstelle 32 mit der Blutpumpe 14 in Verbindung. Die Einheit 30 ist zur Abgabe eines Parameters des Atemzyklus oder eines mit dem Atemzyklus verbundenen Parameters ausgebildet, der über die Ventilationsgeräteschnittstelle 31 an die Steuereinheit 16 übertragen wird, damit die Steuereinheit 16 diesen Parameter zur Steuerung der Blutpumpe 14 über die Blutpumpenschnittstelle 32 verwenden kann.

Der Patient 11 weist als weiteres Gerät ein EKG 33 auf, das über eine EKG-Schnittstelle 34 mit der Steuereinheit 16 in Verbindung steht. Dies ermöglicht es alternativ oder kumulativ zur Ventilationsgeräteschnittstelle 31 über die EKG-Schnittstelle 34 einen Messwert zur Impedanz an die Steuereinheit 16 zu übertragen, damit auch dieser Messwert bei der Steuerung der Blutpumpe 14 über die Blutpumpenschnittstelle 32 berücksichtigt werden kann. An Stelle des EKG 33 kann als Gerät auch ein Myokardsensor verwendet werden, der dann entsprechend über eine Myokardsensor-Schnittstelle einen Messwert zur Muskelaktivität des Zwerchfells an die Steuereinheit 16 überträgt.

Die Figur 3 zeigt eine Anordnung 22 für einen Patienten 12, bei der eine Auswerteeinheit 26 und ein Ventilationsgerät 28 zu einer integrierten Konsole 35 zusammengefasst sind. Diese integrierte Konsole 35 empfängt über die EKG-Schnittstelle 34 Messwerte und sie steht über die Leitung 27 mit dem Patienten 12 in Verbindung. Die in Figur 2 gezeigte Schnittstelle zum Ventilationsgerät 18 kann entfallen, da das Ventilationsgerät 28 mit der Steuereinheit 26 zusammengefasst ist. Die integrierte Konsole 35 kann somit über die Schnittstelle 32 die Blutpumpe 24 steuern, die über den Gasaustauscher 25 und die Kanüle 23 mit dem Patienten 12 in Verbindung steht.

Die Konsole 35 ist dafür ausgelegt, die Triggerung aus dem vorgegebenen mechanischen Atemzyklus zu beziehen. Ein weiteres Gerät ist daher optional. Da Patienten auf der Intensivstation in der Regel an ein EKG angeschlossen sind, bietet es sich an, dieses Signal als zweiten Input zu verwenden.

Wenn ein Patient mit einem Tubus in der Luftröhre beatmet aus dem künstlichen Koma aufwacht und spontan selbst atmet, erkennt die Steuereinheit eine Zustandsänderung und ändert automatisch die Steuerung der Pumpe bzw. den "Geräteeingang". In diesem Fall kann über einen zweiten Sensoreingang beispielsweise das Signal eines EKG verwendet werden, um den Durchfluss an der Blutpumpe 14, 24 zu steuern.

Dies ermöglicht es auf besonders einfacher Art und Weise mittels der integrierten Konsole 35 den Durchfluss an der Blutpumpe 24 und den Gasfluss am Ventilationsgerät 28 und insbesondere den Algorithmus, also den "Puls" der Blutpumpe und den Zyklus der Atmung zu steuern.

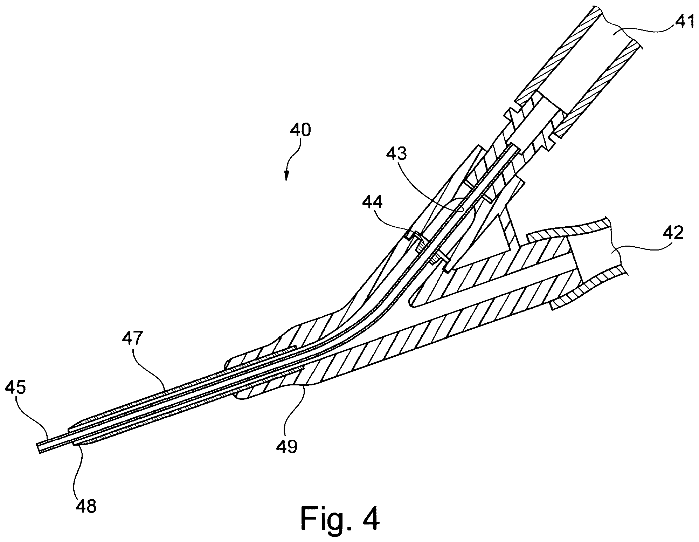

Die Figur 4 zeigt als Beispiel eine Doppellumenkanüle 40 aus der EP19780300235 mit einem ersten Flüssigkeitsanschluss 41 und einem zweiten Flüssigkeitsanschluss 42. Somit gelangt im Einsatz Flüssigkeit durch die Kanüle 43, die durch die Dichtung 44 geführt ist, bis zur Kanülenspitze 45. Ein zweiter Flüssigkeitsstrom gelangt vom Kanülenbereich 48 über die Leitung 46 durch die radial äußere koaxiale Kanüle 47, die im I<anülenkörper 49 gehalten ist, bis zum Kanüleneingang 48.

Sowohl an der Kanülenspitze 45 als auch am Kanüleneingang 48 kann sich eine Wandung des Gefäßes, in das die Kanüle geschoben wurde, an die Kanüle anlegen und die Strömung behindern. Besonders gefährdet ist der Bereich 48 der Kanüle 40, an dem Flüssigkeit wie insbesondere Blut abgesaugt wird.

Wenn mit einer einfachen Kanüle (z.B. venöse Fernoralkanüle) Flüssigkeit aus einem Gefäß abgezogen wird, dann ist die Kanülenspitze derjenige Bereich, der besonders gefährdet ist.

## Patentansprüche

1. Anordnung (2, 12, 22) zur extrakorporalen Lungen- und Herzunterstützung mit einer Blutpumpe (4, 14, 24), einem Gasaustauscher (5, 15, 25), einer Steuereinheit (6, 16, 26), um den Durchfluss an der Blutpumpe (4, 14, 24) zu steuern und den optimalen Zeitpunkt der Blutentnahme an einem Patienten in Abhängigkeit von der Atmung des Patienten abzupassen und den Blutfluss entsprechend einzustellen, einer Kanüle (3, 13, 23), mit der vom Patienten Blut entnommen werden kann, das mit der Blutpumpe (4, 14, 24) durch den Gasaustauscher (5, 15, 25) gefördert wird, wobei die Kanüle mit der Blutpumpe (4, 14, 24) in Verbindung steht, sodass Blut mit der Blutpumpe (4, 14, 24) durch den Gasaustauscher (5, 15, 25) gefördert werden kann, und mindestens einem Gerät (8, 30, 33), um Messwerte an die Steuereinheit (6, 16, 26) zu übermitteln, ***dadurch gekennzeichnet, dass*** das Gerät (8, 30, 33) zur Abgabe eines Parameters des Atemzyklus oder mit dem Atemzyklus verbundenen Parameters ausgebildet ist.

2. Anordnung nach Anspruch 1, ***dadurch gekennzeichnet, dass*** die Blutpumpe (4) einen Antrieb aufweist, um zur Variation des Durchflusses dessen Leistung zu steuern.

3. Anordnung nach einem der vorhergehenden Ansprüche, ***dadurch gekennzeichnet, dass*** die Blutpumpe (4, 14, 24) einen Rotor aufweist, um zur Variation des Durchflusses dessen Drehzahl zu steuern.

4. Anordnung nach einem der vorhergehenden Ansprüche, ***dadurch gekennzeichnet, dass*** das Gerät ein EKG (33) ist und der Parameter mit einer Impedanz des EKG korreliert.

5. Anordnung nach einem der vorhergehenden Ansprüche, ***dadurch gekennzeichnet, dass*** das Gerät ein Ventilationsgerät (8, 18, 28) ist und der Parameter mit einem Inspirationsdruck des Ventilationsgeräts (8, 18, 28) korreliert.

6. Anordnung nach einem der vorhergehenden Ansprüche, ***dadurch gekennzeichnet, dass*** das Gerät ein Brustgurt ist und der Parameter mit einer Dehnung oder Spannung am Brustgurt korreliert.

7. Anordnung nach einem der vorhergehenden Ansprüche, ***dadurch gekennzeichnet, dass** das* Gerät ein Piezoelement ist.

8. Anordnung nach einem der vorhergehenden Ansprüche, ***dadurch gekennzeichnet, dass*** das Gerät ein Zwerchfell- oder Myokardsensor ist.

9. Anordnung nach einem der vorhergehenden Ansprüche, ***dadurch gekennzeichnet, dass*** das Gerät eine sensorische Magensonde ist.

10. Anordnung nach einem der vorhergehenden Ansprüche, ***dadurch gekennzeichnet, dass*** die Steuereinheit (6, 16, 26) Blutfluss- und Gasflussparameter eines Gasaustauschers (5, 15, 25) und ein Ventilationsgerät (8, 18, 28) überwacht.

11. Anordnung nach einem der vorhergehenden Ansprüche, ***dadurch gekennzeichnet, dass*** die Steuereinheit (6, 16, 26) auf der Grundlage von vorher eingegebenen Daten Messwerte von unterschiedlichen Geräten (8, 18, 28, 33) auswählt oder kombiniert, um den Durchfluss an der Blutpumpe (4, 14, 24) zu steuern.

12. Anordnung nach einem der vorhergehenden Ansprüche, ***dadurch gekennzeichnet, dass*** nach der Blutpumpe (4, 14, 24) ein Pufferelement angeordnet ist.

13. Anordnung nach einem der vorhergehenden Ansprüche, ***dadurch gekennzeichnet, dass*** die Blutpumpe (4, 14, 24) mit einer Doppellumenkanüle in Verbindung steht.

## Claims

1. An arrangement (2, 12, 22) for extracorporeal lung and heart support, comprising a blood pump (4, 14, 24), a gas exchanger (5, 15, 25), a control unit (6, 16, 26) for controlling the flow at the blood pump (4, 14, 24) and for matching the optimum time for blood withdrawal from a patient in dependence on the patient's breathing and for adjusting the blood flow accordingly, a cannula (3, 13, 23) with which blood can be withdrawn from the patient, which blood is conveyed by the blood pump (4, 14, 24) through the gas exchanger (5, 15, 25), the cannula being in communication with the blood pump (4, 14, 24) so that blood can be delivered through the gas exchanger (5, 15, 25) by means of the blood pump (4, 14, 24), and at least one device (8, 30, 33) for transmitting measured values to the control unit (6, 16, 26), ***characterized in that** the* device (8, 30, 33) is adapted to output a parameter of the respiratory cycle or a parameter associated with the respiratory cycle.

2. The arrangement according to claim 1, ***characterized in that*** the blood pump (4) has a drive for controlling the output thereof to vary the flow rate.

3. The arrangement according to any one of the preceding claims, ***characterised in that*** the blood pump (4, 14, 24) comprises a rotor for controlling the speed thereof to vary the flow rate.

4. The arrangement according to any one of the preceding claims, *characterized in thatthe* device is an ECG (33) and the parameter correlates with an impedance of the ECG.

5. The arrangement according to any one of the preceding claims, ***characterized in that*** the device is a ventilation device (8, 18, 28) and the parameter correlates with an inspiratory pressure of the ventilation device (8, 18, 28).

6. The arrangement according to any one of the preceding claims, ***characterised in that** the* device is a chest strap and the parameter correlates with a stretch or tension on the chest strap.

7. The arrangement according to any one of the preceding claims, *characterised in thatthe* device is a piezoelectric element.

8. The arrangement according to any one of the preceding claims, *characterised in thatthe* device is a diaphragm or myocardial sensor.

9. The arrangement according to any one of the preceding claims, *characterised in thatthe* device is a sensory gastric tube.

10. The arrangement according to any one of the preceding claims, ***characterized in that** the* control unit (6, 16, 26) monitors blood flow and gas flow parameters of a gas exchanger (5, 15, 25) and a ventilation device (8, 18, 28).

11. The arrangement according to any one of the preceding claims, ***characterised in that** the* control unit (6, 16, 26) selects or combines measured values from different devices (8, 18, 28, 33) on the basis of previously input data in order to control the flow at the blood pump (4, 14, 24).

12. The arrangement according to one of the preceding claims, ***characterised in that*** a buffer element is arranged downstream the blood pump (4, 14, 24).

13. The arrangement according to any one of the preceding claims, ***characterized in that** the* blood pump (4, 14, 24) is in communication with a double lumen cannula.

## Revendications

1. Agencement (2, 12, 22) pour l'assistance pulmonaire et cardiaque extracorporelle avec une pompe à sang (4, 14, 24), un échangeur de gaz (5, 15, 25), une unité de commande (6, 16, 26), afin de commander le débit au niveau de la pompe à sang (4, 14, 24) et d'attendre le moment optimal pour le prélèvement de sang chez un patient en fonction de la respiration du patient et de régler de manière correspondante le débit sanguin, une canule (3, 13, 23), par laquelle du sang peut être prélevé du patient, lequel sang est transporté par la pompe à sang (4, 14, 24) à travers l'échangeur de gaz (5, 15, 25), dans lequel la canule est en communication avec la pompe à sang (4, 14, 24) de façon à ce que du sang puisse être transporté par la pompe à sang (4, 14, 24) à travers l'échangeur de gaz (5, 15, 25), et au moins un appareil (8, 30, 33), afin de transmettre des valeurs de mesure à l'unité de commande (6, 16, 26), **caractérisé en ce que** l'appareil (8, 30, 33) est conçu pour la délivrance d'un paramètre du cycle respiratoire ou d'un paramètre lié au cycle respiratoire.

2. Agencement selon la revendication 1, **caractérisé en ce que** la pompe à sang (4) présente un entraînement, afin de commander la puissance de celui-ci pour faire varier le débit.

3. Agencement selon l'une des revendications précédentes, **caractérisé en ce que** la pompe à sang (4, 14, 24) présente un rotor, afin de commander la vitesse de rotation de celui-ci pour faire varier le débit.

4. Agencement selon l'une des revendications précédentes, **caractérisé en ce que** l'appareil est un ECG (électrocardiographe) (33) et le paramètre est corrélé avec une impédance de l'ECG.

5. Agencement selon l'une des revendications précédentes, **caractérisé en ce que** l'appareil est un appareil de ventilation (8, 18, 28) et le paramètre est corrélé avec une pression inspiratoire de l'appareil de ventilation (8, 18, 28).

6. Agencement selon l'une des revendications précédentes, **caractérisé en ce que** l'appareil est une ceinture pectorale et le paramètre est corrélé avec une dilatation ou une tension au niveau de la ceinture pectorale.

7. Agencement selon l'une des revendications précédentes, **caractérisé en ce que** l'appareil est un élément piézoélectrique.

8. Agencement selon l'une des revendications précédentes, **caractérisé en ce que** l'appareil est un capteur diaphragmatique ou myocardique.

9. Agencement selon l'une des revendications précédentes, **caractérisé en ce que** l'appareil est une sonde gastrique sensorielle.

10. Agencement selon l'une des revendications précédentes, **caractérisé en ce que** l'unité de commande (6, 16, 26) surveille des paramètres de débit sanguin et de débit gazeux d'un échangeur de gaz (5, 15, 25) et un appareil de ventilation (8, 18, 28).

11. Agencement selon l'une des revendications précédentes, **caractérisé en ce que** l'unité de commande (6, 16, 26) sélectionne ou combine, sur la base de données précédemment saisies, des valeurs de mesure de différents appareils (8, 18, 28, 33), afin de commander le débit au niveau de la pompe à sang (4, 14, 24).

12. Agencement selon l'une des revendications précédentes, **caractérisé en ce qu'**après la pompe à sang (4, 14, 24) est disposé un élément tampon.

13. Agencement selon l'une des revendications précédentes, **caractérisé en ce que** la pompe à sang (4, 14, 24) est en communication avec une canule à double lumière.
